# EUROPEAN PATENT SPECIFICATION

(11) **EP 1 266 665 B1**
(45) Date of publication and mention of the grant of the patent: **11.08.2004**
(21) Application number: 01114275.9
(22) Date of filing: 13.06.2001
(51) Int. Cl.: A61L 2/07

(54) **Device for an autoclave**
Vorrichtung zum Sterilisieren
Dispositif pour un autoclave

(43) Date of publication of application: 18.12.2002
(73) Proprietor: Getinge Skärhamn AB, 471 31 Skärhamn (SE)
(72) Inventor: Linder, Gert, 47131 Skärhamn (SE); Wanselin, Johan, 47131 Skärhamn (SE)
(74) Representative: Lindberg, Klas Valter Bo

(56) References cited:
- EP-A- 0 306 779
- EP-A- 0 429 960
- EP-A- 0 663 213
- WO-A-02/42164
- WO-A-97/48420

## Description

### Technical field of the Invention

The present invention relates to a sterilisation chamber for use in a sterilisation device or the like, said chamber being adapted to enclose goods to be sterilised during a sterilisation process according to claim 1.

This invention also relates to a sterilisation device, being provided with a sterilisation chamber, in which a sterilisation process is intended to be performed.

### Background art

Sterilisation devices are today commonly used in several fields, for example health care and medicine. Sterilisation devices, sometimes referred to as autoclaves may be of different sizes, from small devices for use at a medical or dental clinic, to large industrial devices for use in the production of drugs etc. Common for all these different kinds of sterilisation devices are that they comprise a sterilisation chamber, in which goods that shall be sterilised is placed. In the chamber there may be several inlets/outlets for steam, moist, water or the like, depending on the kind of sterilisation that is to be performed in said sterilisation chamber. Furthermore, means fore pressurising and heating the interior space of said chamber are arranged. Traditionally this chamber is made out of a stainless metal material, being a durable construction.

However, there are some drawbacks with the above-described prior art sterilisation chambers. For example, these chambers may be somewhat time consuming to manufacture, since they require welding or the like. Furthermore, a lot of energy is lost when heating the chamber, since some of the added energy is used for heating the chamber material, resulting in an somewhat ineffective sterilisation process.

The document WO-A-0242164 (published 30.05.02 , priority date: 20.11.00) describes a chamber manufactured from a polymeric material and adapted to include goods to be sterilized.

### Summary of the invention

Consequently, the object of the present invention is to provide a simple chamber construction, that meets the set up medical requirements and that is easy to manufacture. A further object of the invention is to provide a chamber in which the sterilisation process may be executed in an energy-effective manner.

These and other objects are achieved in accordance with the invention by a sterilisation chamber as initially described, being characterised in that said chamber has a self supported structure being essentially manufactured from a polymeric material according to claim 1. By this construction, one gains a plurality of advantages. To start with, the polymer material absorb very little heat, resulting in the fact that essentially all supplied energy is used in the sterilisation process instead of heating the chamber walls. In turn, this results in shorter processing times as well as a lower consumption of energy. Further, the above-described construction utilising polymeric materials has the advantage that condensation is less likely to appear on a plastic surface than a metallic one. Thereby, it is possible to achieve dry goods quicker, and with a lower consumption of energy and water. Further, polymeric materials provide for built-in isolation for heat as well as noise, resulting in a quiet autoclaving chamber having a cool exterior, essentially without the need of further isolation. Yet another advantage with the invention is the fact that polymeric materials are resistant to corrosion. In traditional stainless autoclaving chambers corrosion is often a problem, due to the fact that that the cleaning agents used often comprise chloride compounds.

Further advantages with this invention is that the chamber is light, among other things resulting in cheaper transports, and cheap to produce.

In accordance with a preferred embodiment of the invention, said chamber is manufactured from an injection-mouldable material, resulting in a chamber that is simple to produce. Further, a reinforcement material, such as a glass material is preferably included in said injection-mouldable material. Thereby, the material in the chamber gets stronger and more durable for mechanical stress. Suitably, said injection-mouldable material is essentially a polyamide material, being a strong and well-tested material. In order to provide an even stronger chamber a reinforcement material, such as a rowing weave material may be arranged around said injection moulded material, forming an outer layer of said chamber.

In accordance with a second embodiment of the invention, said chamber is manufactured from a composite material. This implies a rational manufacturing as well as a strong and durable chamber construction. Further, said composite material suitably comprises a carbon fibre rowing weave and a concatenating polymer material, being a strong and well-tested construction. Preferably, said concatenating polymer material is an epoxy material. In accordance with an alternative embodiment, said composite material comprises a glass fibre rowing weave and a concatenating polymer material, being a strong and well-tested construction. Preferably, said concatenating polymer material is one of a polyvinyl, isopolyester or orthopolyester material.

Further, said chamber is preferably releasably mountable in said sterilisation device, whereby the chamber may be mounted in the sterilisation device as an easily exchangeable component. Suitably, said chamber is essentially manufactured in one continuous piece. This facilitates the mounting of the chamber in the sterilisation device, as well as simplifies any exchange of the chamber. Further, it is possible to manufacture the chamber without joints or the like. This has several advantages. To start with this reduces the risk of bacterial build up and consequently results in clean, hygienic surfaces within the chamber. Furthermore, the lack of joints in the chamber is advantageous for reducing wear of the chamber. Preferably components, such as inlets and outlets for steam, moist and the like, are integrally formed with said chamber. This does not only facilitate the mounting of the chamber in the sterilisation device, but also reduces the number of components needed. Further, it also results in a smaller risk of mounting some component in an incorrect manner. Preferably, said chamber is further provided with a pair of integrally formed tracks, in which a sealing chamber door may be slidably mounted. Thereby, no hinges or the like are necessary, whereby associated stress on the chamber material at the attachment points of the chamber door is avoided. Furthermore, the number of joints in the interface between the chamber and the door may be held at a minimum.

Furthermore, the above described and other objects are achieved in accordance with the invention by a sterilisation device, being provided with a sterilisation chamber, in which a sterilisation process is intended to be performed, being characterised in that said chamber is as described above.

### Brief description of the drawing

A currently preferred embodiment of the present invention will now be described in closer detail, with reference to the accompanying drawing.

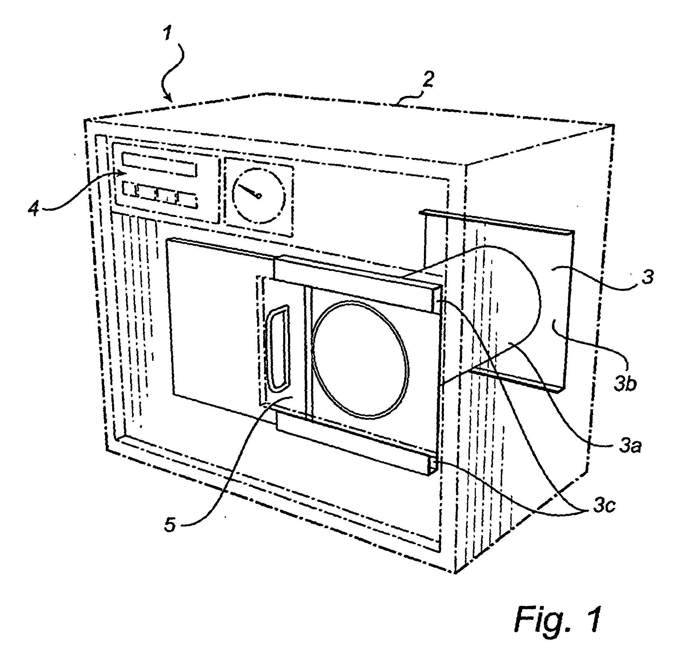

Fig 1 is a perspective view of a sterilisation device, having a sterilisation chamber in accordance with the invention.

### Detailed description of preferred embodiments of the Invention

A sterilisation device 1 is schematically shown in fig 1. The sterilisation device 1 comprises a housing 2, constituting the outer boundary of said sterilisation device 1. Further, the sterilisation device 1 comprises a sterilisation chamber 3, being arranged within said housing 2. Furthermore, the sterilisation device comprises pressure means, vaporisation means etc (not shown) in accordance with prior art devices. These will consequently not be described further herein. The sterilisation device 1 also comprises display means 4, for monitoring and controlling a sterilisation process that is to take place within said chamber 3.

In the shown embodiment of the invention, the above described sterilisation chamber 3 comprises a cylindrical portion 3a having a back wall 3b, together forming a container having a front opening through which goods to be sterilised may be entered. Further, the sterilisation device 1 comprises a chamber door 5, being movable between a first and a second position. In said first position the door is in a closed position in which the door is positioned in front of said front opening and an inside of the door, together with the inside of said sterilisation chamber (i.e. the cylindrical portion and the back wall), creates a sterilisation enclosure. In this position the door is in sealing contact with the chamber. In said second position the door is removed from said front opening, leaving the chamber open, for entering or removing goods to or from said sterilisation chamber. In the shown embodiment the door is slidably arranged between said first and second position.

In accordance with the invention, the chamber 3 is manufactured from a polymeric material. There is a plurality of ways of manufacturing a chamber like this of plastic, for example by injection moulding, casting and so on. The plastic material is so chosen that it is durable for heat and pressure. One advantage with using a polymeric material for the manufacturing of the chamber is that the material has natural isolating properties for heat as well as noise. In traditional autoclaves, a large amount of energy is consumed for heating the material in the metal chamber for each autoclaving cycle. This material heating is eliminated with the inventive construction. Further problems with the chamber heating in traditional sterilisation devices is that the heat may be transported through the chamber walls to the housing, resulting in a risk of burning for the personnel using the device if he or she touches the housing of the device. Consequently, in prior art devices, extra isolation has been needed to avoid this. This problem is also avoided with the inventive construction. Furthermore the sterilisation process, when vapour, water and so on is fed into the chamber on per se known manner, has a tendency to create noise within the chamber. By using the inventive construction, this kind of noise does not leave the chamber due to sound isolating properties of the polymeric material, resulting in an improved work environment for the personnel.

As seen in the picture the sterilisation chamber comprises fastening portions, formed in integration with the rest of the chamber. Said fastening portions 3c are intended to be used for releasable mounting of the chamber in said sterilisation device by means of fasteners (not explicitly shown). In the present case the fastening portions are flat front and back surfaces 3c, provided with openings, through which a screw or the like may be introduced and thereafter fastened in the housing. Other ways of mounting the chamber in the sterilisation device are possible and subject to construction preferences of the skilled man. Consequently, the chamber may easily be removed from the sterilisation device, and may thereby be replaced, in case of wear or altered user needs.

In order to facilitate mounting, inlets for vapour, water and the like, and outlets for excess fluids, are integrally formed in said chamber. Thereby the mounting and the exchange of the chamber are further facilitated. The placement and configuration of inlets and outlets in said chamber are subject to preferences of the skilled man, and previously known, and will not be closer described herein.

As shown in fig 1, the chamber door 5 is slidably mounted in a pair of parallel tracks 3c, being formed in integration with said chamber. Said tracks encompass the door on two opposite edges. Further, sealing means are provided on the door in order to provide for the creation of a sealed pressure chamber when the door is in said closed position. By arranging the door as a slidable component no hinges or the like need to be fastened on said chamber, which could result in high wear in the hinge fastening points. By utilising the above described track solution, such wear is avoided, resulting in a longer working life of the chamber.

Furthermore, an insert (not shown) for holding trays or the like may be arranged within said chamber on per se known manner.

According to a first embodiment of the invention, the chamber 3 is manufactured in one piece using injection moulding. The manufacturing material is an injection-mouldable polymeric material, in this case a polyamide material, although other materials are feasible. A reinforcement material is added to said polymeric material in order to create a strong chamber structure. Said reinforcement material may be glass fibres or the like.

According to a second embodiment of the invention, the chamber is constructed from a composite material, i.e. a material having two or more self-supporting structures. The composite material may for example be a rowing weave of carbon fibre, together with an epoxy material or a rowing weave of glass fibre, together with a polyvinyl, isopolyester or orthopolyester material, depending on requirements on strength and cost.

In the shown embodiment of the invention, the chamber is manufactured in one piece, i.e. without joints. This results in a clean and smooth inner surface of the chamber, resulting in a decreased risk for bacterial buildups. Furthermore, due to material properties, condensation is less likely to appear on plastic material than on metallic materials. Consequently, it is easier to achieve dry goods in the chamber, since the condensation moist droplets are fewer, whereby the added energy and may be used in the sterilisation process instead of vaporising said droplets.

The present invention should not be considered as being limited to the above-described embodiments, but rather includes all possible variations covered by the scope defined by the appended claims.

For example, the above-described sterilisation device is fed from the side. However, the invention is naturally not limited to this kind of device, but also includes top-fed devices, feed-through devices and so on. Furthermore, the above-described device is mainly a rather small autoclave, for use in a dentist clinic or the like, but it goes without saying that the inventive construction may be used in various application, from small clinic devices to large industrial autoclave applications. However, for each case, the chamber needs to be adjusted to fit the present demands regarding temperature and pressure durability.

Furthermore, the chamber as shown in the figure has a cylindrical shape with an essentially circular cross section. However, other shapes, such as oval or essentially rectangular shapes are possible and suitable for certain applications. In this case, consideration needs to be taken to the mechanical strength of the construction. Furthermore, although the above-described embodiment refer to certain presently preferred material choices, it goes without saying that other materials, fulfilling the corresponding demands on durability, are equally usable.

It shall also be noted that the chamber may be produced in several pieces, subsequently mounted together to form said chamber, although the above-described "one-piece"-chamber is preferred in most applications. In order to form a chamber from several separate pieces, the pieces may be laminated by hand and thereafter put together by means of plastic welding or the like. This procedure does not require the use of a moulding tool, and may therefor be preferred when a small number of chambers shall be produced.

## Claims

1. Sterilisation chamber for use in a sterilisation device or the like, which sterilisation chamber is arranged to be mounted to the remaining sterilisation device and is arranged to partly define a sterilisation enclosure in said sterilisation device, said chamber being adapted to enclose goods to be sterilised during a sterilisation process, **characterised in that** said chamber has a self supported structure being essentially manufactured from a polymeric material.

2. Sterilisation chamber according to claim 1, wherein said chamber is manufactured from an injection-mouldable material.

3. Sterilisation chamber according to claim 2, wherein a reinforcement material, such as a glass material is included in said injection-mouldable material.

4. Sterilisation chamber according to claim 2 or 3, wherein a reinforcement material, such as a rowing weave material is arranged around said injection moulded material, forming an outer layer of said chamber.

5. Sterilisation chamber according to claim 2, 3 or 4 wherein said injection-mouldable material essentially is a polyamide material.

6. Sterilisation chamber according to claim 1, wherein said chamber is manufactured from a composite material.

7. Sterilisation chamber according to claim 6, wherein said composite material comprises a carbon fibre rowing weave and a concatenating polymer material.

8. Sterilisation chamber according to claim 7, wherein said concatenating polymer material is an epoxy material.

9. Sterilisation chamber according to claim 6, wherein said composite material comprises a glass fibre rowing weave and a concatenating polymer material.

10. Sterilisation chamber according to claim 9, wherein said concatenating polymer material is one of a polyvinyl, isopolyester or orthopolyester material.

11. Sterilisation chamber according to any one of the preceding claims, wherein said chamber is releasably mountable in said sterilisation device.

12. Sterilisation chamber according to any one of the preceding claims, wherein said chamber is essentially manufactured in one continuous piece.

13. Sterilisation chamber according to claim 12, wherein components, such as inlets and outlets for steam, moist and the like, are integrally formed with said chamber.

14. Sterilisation chamber according to claim 12 or 13, wherein said chamber is provided with a pair of integrally formed tracks, in which a sealing chamber door may be slidably mounted.

15. Sterilisation device, being provided with a sterilisation chamber, in which a sterilisation process is intended to be performed, **characterised in that** said chamber is as described in any one of the claims 1-14.

## Patentansprüche

1. Sterilisationskammer zur Verwendung in einer Sterilisationsvorrichtung oder dergleichen, wobei diese Sterilisationskammer zum Einbau in den übrigen Teil der Sterilisationsvorrichtung vorgesehen ist und so konfiguriert ist, dass sie teilweise ein Sterilisationsgehäuse in der Sterilisationsvorrichtung definiert, wobei diese Kammer so beschaffen ist, dass sie Gegenstände, die im verlauf eines Sterilisationsvorganges sterilisiert werden sollen, umschließt, **dadurch gekennzeichnet, dass** diese Kammer eine selbsttragende Konstruktion aufweist, die im wesentlichen aus einem Polymermaterial hergestellt ist.

2. Sterilisationskammer nach Anspruch 1, wobei diese Kammer aus einem spritzgussfähigen Material hergestellt ist.

3. Sterilisationskammer nach Anspruch 2, wobei ein Verstärkungsmaterial wie beispielsweise ein Glasmaterial in das spritzgussfähige Material eingearbeitet ist.

4. Sterilisationskammer nach Anspruch 2 oder 3, wobei ein Verstärkungsmaterial wie beispielsweise ein Rovinggewebematerial um das spritzgegossene Material herum angeordnet ist und so eine äußere Schicht der Kammer bildet.

5. Sterilisationskammer nach Anspruch 2, 3 oder 4, wobei das spritzgussfähige Material im wesentlichen ein Polyamidmaterial ist.

6. Sterilisationskammer nach Anspruch 1, wobei diese Kammer aus einem Verbundmaterial hergestellt ist.

7. Sterilisationskammer nach Anspruch 6, wobei das Verbundmaterial ein Kohlefaser-Rovinggewebe und ein verkettendes Polymermaterial umfasst.

8. Sterilisationskammer nach Anspruch 7, wobei das verkettende Polymermaterial ein Epoxidmaterial ist.

9. Sterilisationskammer nach Anspruch 6, wobei das Verbundmaterial ein Glasfaser-Rovinggewebe und ein verkettendes Polymermaterial umfasst.

10. Sterilisationskammer nach Anspruch 9, wobei das verkettende Polymermaterial entweder ein Polyvinyl-, ein Isopolyester- oder ein Orthopolyestermaterial ist.

11. Sterilisationskammer nach einem der vorangehenden Ansprüche, wobei die Kammer herausnehmbar in der Sterilisationsvorrichtung angeordnet werden kann.

12. Sterilisationskammer nach einem der vorangehenden Ansprüche, wobei die Kammer im wesentlichen aus einem ganzen Stück hergestellt ist.

13. Sterilisationskammer nach Anspruch 12, wobei Komponenten wie beispielsweise Einlässe und Auslässe für Dampf, Feuchtigkeit oder dergleichen integral mit der Kammer ausgebildet sind.

14. Sterilisationskammer nach Anspruch 12 oder 13, wobei diese Kammer mit einem Paar integral ausgebildeter Schienen versehen ist, in die eine abdichtende Kammertür gleitend eingesetzt werden kann.

15. Sterilisationsvorrichtung, die mit einer Sterilisationskammer ausgestattet ist, in der ein Sterilisationsvorgang ausgeführt werden soll, **dadurch gekennzeichnet, dass** die Kammer nach einem der Ansprüche 1-14 beschaffen ist.

## Revendications

1. Chambre de stérilisation pouvant être utilisée dans un dispositif de stérilisation ou similaires, laquelle chambre de stérilisation est agencée de façon à être fixée au dispositif de stérilisation restant et est agencée de façon à définir partiellement une enceinte de stérilisation dans ledit dispositif de stérilisation, ladite chambre étant adaptée pour contenir des éléments devant être stérilisés au cours d'un procédé de stérilisation, **caractérisée en ce que** ladite chambre présente elle-même une structure autosupportée étant essentiellement fabriquée à partir d'un matériau polymère.

2. Chambre de stérilisation selon la revendication 1, dans laquelle ladite chambre est fabriquée à partir d'un matériau pouvant être moulé par injection.

3. Chambre de stérilisation selon la revendication 2, dans laquelle un matériau de renforcement, tel qu'un matériau en verre, est compris dans ledit matériau pouvant être moulé par injection.

4. Chambre de stérilisation selon la revendication 2 ou 3, dans laquelle un matériau de renforcement, tel qu'un matériau provenant du tissage de stratifils, est agencé autour dudit matériau moulé par injection, formant une couche externe de ladite chambre.

5. Chambre de stérilisation selon la revendication 2,3 ou 4, dans laquelle ledit matériau pouvant être moulé par injection est essentiellement un matériau polyamide.

6. Chambre de stérilisation selon la revendication 1, dans laquelle ladite chambre est fabriquée à partir d'un matériau composite.

7. Chambre de stérilisation selon la revendication 6, dans laquelle ledit matériau composite comprend un tissage de stratifils en fibre de carbone et un matériau polymère obtenu par concaténation.

8. Chambre de stérilisation selon la revendication 7, dans laquelle ledit matériau polymère obtenu par concaténation est un matériau époxy.

9. Chambre de stérilisation selon la revendication 6, dans laquelle ledit matériau composite comprend un tissage de stratifils en fibre de verre et un matériau polymère obtenu par concaténation.

10. Chambre de stérilisation selon la revendication 9, dans lequel ledit matériau polymère obtenu par concaténation est un matériau à base de polyvinyle, de polyester iso ou de polyester ortho.

11. Chambre de stérilisation selon l'une quelconque des revendications précédentes, dans laquelle ladite chambre peut être solidement fixée dans ledit dispositif de stérilisation.

12. Chambre de stérilisation selon l'une quelconque des revendications précédentes, dans laquelle ladite chambre est essentiellement fabriquée en une pièce continue.

13. Chambre de stérilisation selon la revendication 12, dans laquelle les composants, tels que les orifices d'entrée et les orifices de sortie pour la vapeur d'eau, l'humidité et similaires, sont intégralement formés avec ladite chambre.

14. Chambre de stérilisation selon la revendication 12 ou 13, dans laquelle ladite chambre est équipée d'une paire de rails intégralement formés, sur lesquels une porte pour chambre étanche peut être montée sur glissière.

15. Dispositif de stérilisation étant équipé d'une chambre de stérilisation, dans lequel un procédé de stérilisation doit être réalisé, **caractérisé en ce que** ladite chambre est telle que décrite dans l'une quelconque des revendications 1 à 14.
